# EUROPEAN PATENT SPECIFICATION

(11) **EP 1 833 385 B1**
(45) Date of publication and mention of the grant of the patent: **27.08.2008**
(21) Application number: 05811830.8
(22) Date of filing: 07.12.2005
(51) Int. Cl.: A61B 17/16, A61F 2/46

(54) **SURGICAL TOOL HOLDER FOR FACILITATED STERILIZATION**
OPERATIONSINSTRUMENTENHALTER FÜR LEICHTERE STERILISATION
PORTE-INSTRUMENT CHIRURGICAL POUR STERILISATION FACILITEE

(30) Priority: 09.12.2004 US 634467 P; 24.08.2005 US 710845 P
(43) Date of publication of application: 19.09.2007
(73) Proprietor: PRECIMED S.A., 2534 Orvin (CH)
(72) Inventor: BURGI, Jonas, CH-2740 Moutier (CH); LECHOT, Andre, CH-2534 Orvin (CH); DESARZENS, Yves, CH-2606 Corgémont (CH)
(74) Representative: Mötteli-Mantelli, Novella
(86) International application number: PCT/IB2005/003720
(87) International publication number: WO 2006/061708

(56) References cited:
- US-A- 5 720 750

## Description

### Background of the Invention

This invention relates to surgical tools for aiding in the installation of orthopedic prostheses in patients. More particularly, the present invention relates to easily sterilizable holders for use with a surgical tool in preparing a bone site, and for use in installing a prosthesis in the bone.

Complicated mechanical devices have crevices and recesses that are difficult, if not almost impossible to clean with ease without disassembly into separate component parts. Devices that are not properly cleaned and sterilized increase the risk of disease transfer from patient to patient. This is especially true following the emergence of certain "prions" that are not killed by normal hospital sterilization.

Further, in surgical procedures in which access to the treatment site is limited, it is difficult to use current solutions without subjecting the patient to repeated abrasion and tissue trauma when inserting and extracting surgical instruments.

Additionally, the insertion of a prosthetic implant is often problematic, and the orientation of the implant in a properly prepared recess is often critical to minimize recovery time of the patient. Still further, once the appropriate position of the tool is selected, it is often difficult to ensure that the position does not change upon insertion of the assembly through the incision.

US5720750 discloses an orthopedic tool holdes according to the preamble of claim 1. It would be beneficial, therefore, to have an orthopedic tool holder that is easily adjustable, disassemblable, and cleanable. Additionally, it would be beneficial if the tool holder can be partially disassembled for cleaning without the need to completely separate any of the component parts completely from the device as a whole. Further, it would be beneficial to have a holder that enables the surgeon to better maneuver and position a tool head to facilitate preparing a bone site to receive a prosthetic implant in a particular angular orientation.

### Summary of the Invention

A surgical tool holder aids a surgeon in controlling the use of a tool during surgery, for example, during preparation of a femoral cavity for reception of hip joint prosthesis. The present invention is such a surgical tool holder, but adapted to facilitate sterilization. The adaptation is a "break-away" feature that additionally provides a tool holder that allows partial disassembled to facilitate sterilization, while remaining loosely intact to prevent the separation of component parts from the device as a whole.

The present surgical holder has a housing which encloses a break-away, surgical tool engagement mechanism. At one end, the present tool holder has a tool or prosthesis engaging interface. At the opposite end, the holder has a handle or grip which facilitates manipulation of the holder and positioning of an attached tool during use. The holder enables easy orientation of the surgical tool or other device attached to its interface. This is important, because control is critical in order to accurately prepare a bone site for receiving a prosthesis, and for installing a prosthesis in the site.

An objective of the present invention is to be easily cleaned and sterilized. This is accomplished by the holder including a tool head engagement mechanism that easily breaks-away (unfolds) from the rest of the device, but remains attached as a module. This object of the invention eliminates the need to disassemble the tool holder into separated pieces, and minimises the risk that pieces could be lost. The disassembled/unfolded condition allows access to all surfaces to be cleaned and sterilized. Cleaning and sterilization is further facilitated by the holder having a reduced the number of small radius internal corners, crevices and small gaps, and by the absence of blind holes. Another object of the invention is to provide a tool holder which enables an attached tool or prosthesis to be firmly locked against rotation or rocking.

### Brief Description of the Drawings

Figs. 1A and 1B are side view schematics illustrating the present surgical tool holder in an assembled/ready-to-use condition (A), and in a disassembled/unfolded condition for cleaning and/or sterilization.
Fig. 2A is a partial cross-sectional side view of a surgical tool holder of the present invention illustrating the tool holder mechanism in an "open" configuration, i.e., ready to receive a surgical or orthopedic tool for attachment to the handle.
Fig. 2B is a partial cross-sectional side view of a surgical tool holder of the present invention illustrating the tool holder mechanism in a "closed" configuration and a surgical tool (a broach) attached to the handle.
Fig. 3A is a side view of a tool holder linkage of the present invention.
Fig. 3B is an end-on view of an engagement lever of the tool holder linkage of Fig. 3A.
Fig. 4 is a partial cross-sectional side view of the housing of the present tool holder showing the tethered relationship of the tool holder linkage to the housing.
Figs. 5A, 5B and 5C are side views of a portion of the housing illustrating alternative releasable locking mechanisms for positively retaining the tool holder linkage within the housing.
Fig. 6 is a partial cross-sectional side view of a surgical tool holder of the present invention illustrating an alternative configuration of the tool holder mechanism including an engagement lever locking feature.
Fig. 7A is a partial cross-sectional side view through the housing of the tool holder in an alternative embodiment of the present invention.
Fig. 7B is a side view of an embodiment of the present surgical tool holder showing the tool holder linkage assembly retained in the housing.
Fig, 7C is a perspective view of the present tool holder illustrating an example of a one-way catch mechanism.
Fig. 8A is side view of an embodiment of the present surgical tool holder illustrating the operational feature of the tool holder linkage assembly and its relationship to an attached tool.
Fig. 8B is a perspective view of the present surgical tool holder illustrating the use of the one way locking mechanism to lock an installed attachment against the holder's housing head.
Figs. 9A to 9D are perspective views of an embodiment of the present tool holder showing various stages of disassembly for cleaning and reassembly for use.
Figs. 10A to 10D are partial cross-sectional side views of an embodiment of the present surgical tool holder illustrating a further alternative releasable locking mechanism for positively retaining the tool holder drive chain linkage within the housing.
Fig. 11 is a schematic view of a prior art tool holder.
Fig. 12 is a schematic view of the surgical tool holder of the invention in operation.

### Detailed Description of the Preferred Embodiment(s)

The present invention is a surgical tool holder configured for facilitated cleaning and sterilization. Various surgical/orthopedic tool heads useful for surgical procedures can be mounted on the tool holder. The present surgical tool holder includes a tool holder linkage having an interface for attaching a tool head to the holder. The tool holder linkage is partially removable from the body of the surgical tool holder to facilitate cleaning and sterilization of the device as a whole. The tool holder linkage intentionally is only partially removable from the body of the handle to prevent its component parts from being separated the device as a whole, for example during cleaning, handling or storage, and subsequently becoming lost or misplaced.

Referring now to the drawings, the details of preferred embodiments of the present invention are graphically and schematically illustrated. Like elements in the drawings are represented by like numbers, and any similar elements are represented by like numbers with a different lower case letter suffix.

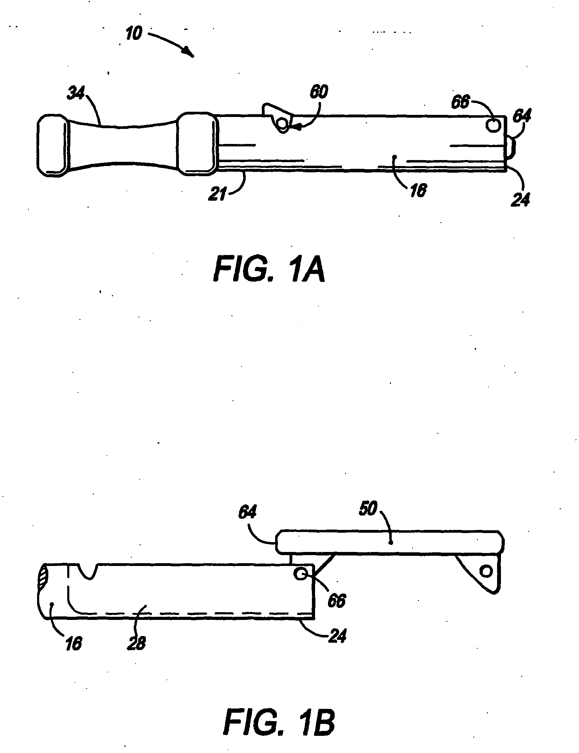

The present invention is a break-away surgical tool holder 10 as generally exemplified in Figs. 1A and 1B. As such, the present surgical tool handle 10 includes all of the materials composition limitations expected of such a tool for surgical use and subject to sterilization procedures. Additionally, the present surgical tool holder 10 includes a tethered tool holder linkage assembly that has new structural features and functional aspects that distinguish it from prior surgical tool holders. More specifically, the tool holder 10 has a break-away tool holder linkage assembly 50 that allows the tool holder linkage 50, which during use is normally retained in the housing 16 of the holder 10 in a linkage chamber 28 (see Fig. 1A), to be released and swung or folded out of the housing 16 (see Fig. 1B) for cleaning. An important feature of the tool holder 10 is that when disassembled to this folded out condition, cleaning and sterilization of the holder 10 is facilitated, without any of the component parts of the holder 10 having to be separated from the device as a whole. As noted above, this feature prevents the loss of any parts of the holder during cleaning/sterilization or during use in a surgical procedure.

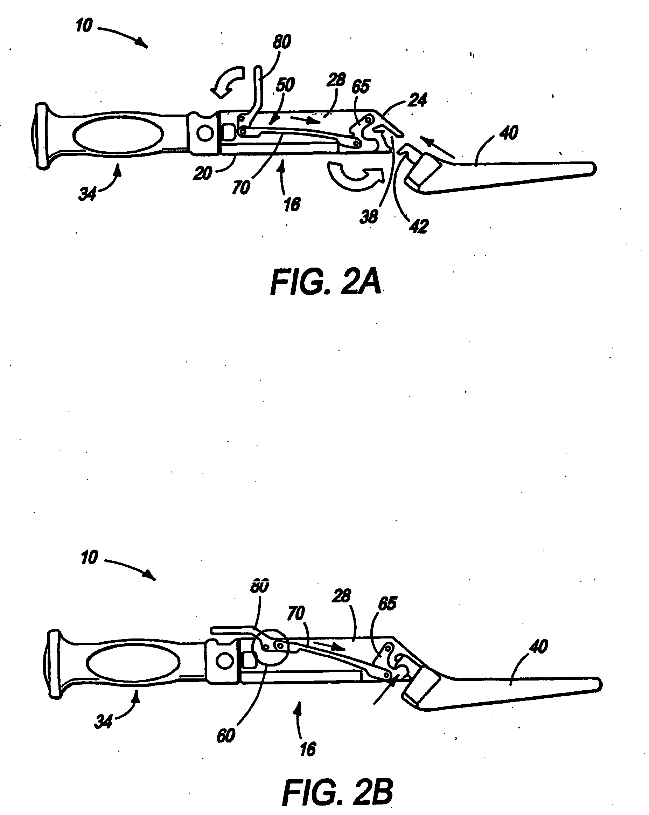

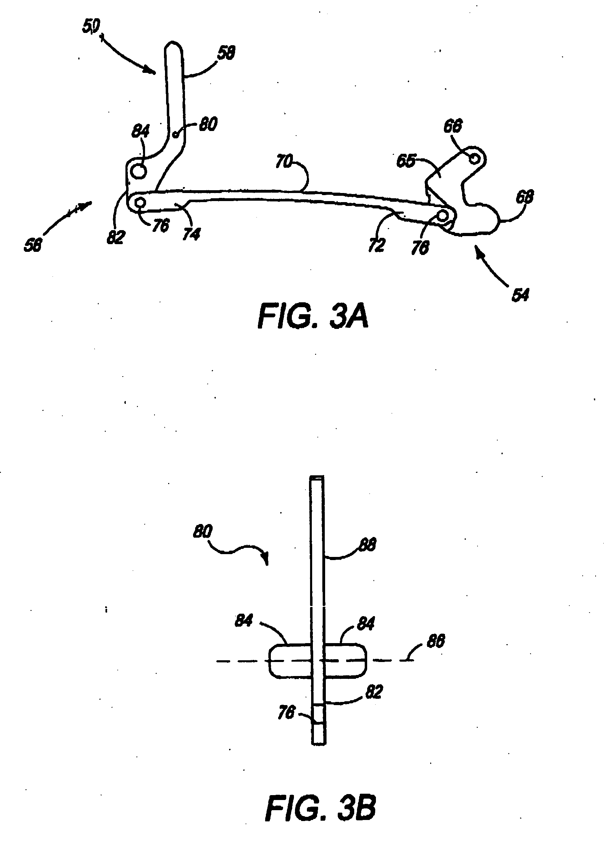

In a preferred embodiment illustrated in Figs. 2A and 2B, the body of the surgical tool holder 10 includes an elongated housing 16, having a grip end 20, a tool end 24, and a linkage chamber 28. A hand grip 34 is attached to the housing 16 at the grip end 20. A tool insertion port 38 is disposed at the tool end 24 of the housing 16 for receiving a surgical tool head 40, such as a surgical rasp. A tool holder linkage 50 is received within the linkage chamber 28. The linkage 50 allows a tool head 40 to be attached to or removed from the tool end 24 of the surgical tool holder 10. The tool holder linkage 50 is tethered at its tool holder end 54 (see Fig. 3A) to the interior of the linkage chamber 28 as described below. The other end or the linkage retainer end 56 (see Fig. 3A) of the linkage 50 communicates with a linkage lock mechanism 60 to hold the tool holder linkage 50 within the linkage chamber 28 and to secure the attachment of the tool head 40 to the housing 16.

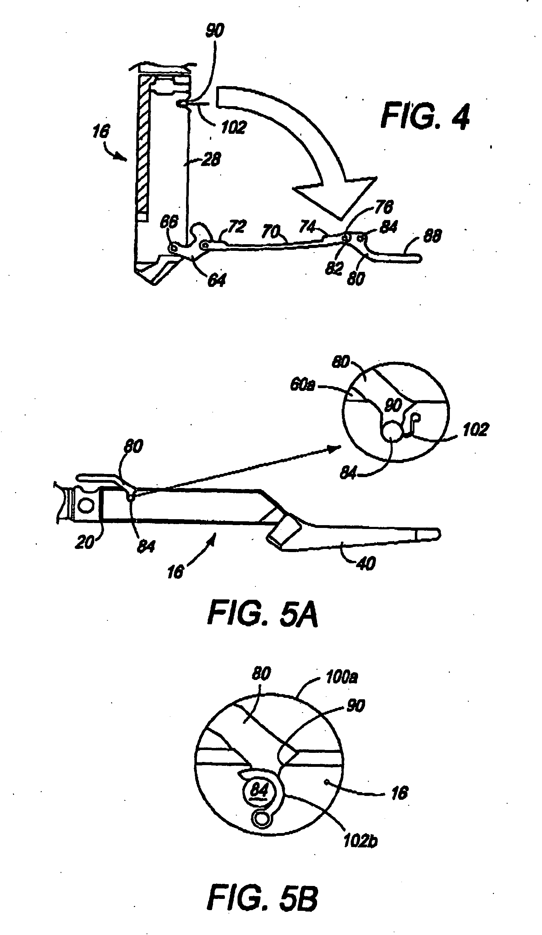

The tool linkage 50 is further illustrated in Fig. 3A. The linkage 50 has a tool holder end 54 and a linkage retainer end 56. A tool interface 64, comprising a locking pawl 65 and the tool port 38 in this embodiment, is disposed at the tool holder end 54 of the linkage 50. The locking pawl 65 swivels about a pivot pin 66. The pivot pin 66 tethers the entire tool holder linkage 50 to the interior of the linkage chamber 28, allowing the rest of the linkage 50 to be swung out of the chamber 28 to facilitate cleaning/sterilization without any of the component parts of the device having to be separated from the device as a whole. See Fig. 4.

As shown in Figs. 3A and 3B, the locking pawl 65 is in driven communication with the drive end 72 of a torsion connecting rod 70 via a mechanical movement coupling means 76. The drive end 72 of the torsion rod 70 causes the locking pawl 65 to swivel on pivot pin 66 in response to movement of the torsion rod 70. The torsion connecting rod 70 has a driven end 74 in communication with the lever follower arm 82 of an engagement lever 80 at the linkage retainer end 56 of the tool holder linkage 50.

In the preferred embodiment illustrated in Fig. 3B, the engagement lever 80 has a pair of opposed lever pins 84 centered on a engagement lever axis of rotation 86 about which axis 86 the engagement lever 80 is pivotable. The elongated housing 16 includes a pair of catch recesses 90 (see Fig.4), each catch recess 90 disposed to pivotably receive a lever pin 84 of the pair of lever pins to mount the engagement lever 80 to the housing 16. The engagement lever 80 is pivotable when mounted in the catch recesses 90 so that moving the lever handle 88 of the engagement lever 80 brings the follower arm 82 to bear against the drive end 74 of the torsion connecting rod 70. In the preferred embodiment shown, the movement coupling 76 used at either end of the torsion rod 70 was a simple swivel connector, such as is known to the ordinary skilled artisan in view of the illustrations. Other coupling means are know to and selectable by one of skill in the art for practice in the present invention as well.

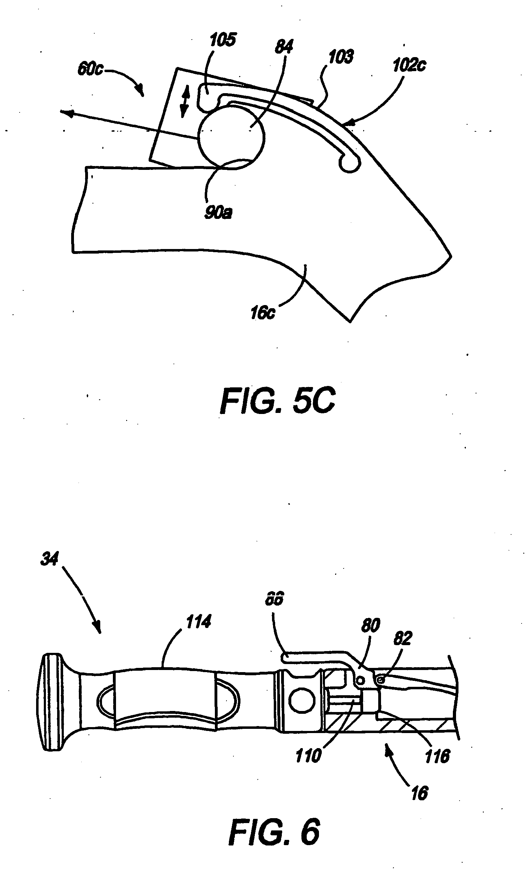

As shown in Fig. 5A, a linkage lock mechanism 60a was disposed proximate the grip end 20a of the housing 16a. The linkage lock mechanism 60a engaged the engagement lever 80 to retain the tool holder linkage 50 in the linkage chamber 28. In a preferred embodiment of the linkage lock mechanism 60a illustrated in Fig. 5A, one of the lever pins 84 of the engagement lever 80 is engaged to retain the tool holder linkage 50a in the linkage chamber 28a. In the illustrated embodiment, a detent bias 102 associated with a catch recess 90 normally bears against a lever pin 84 received in the catch recess 90 to hold the lever pin 84 in place and retain the holder linkage 50 in the linkage camber 28. In the embodiment illustrated, the detent bias 102 bearing against the lever pin 84 was integral to and inseparable from the housing 16. Other locking mechanisms are available as well. For example, in Fig. 5B, the linkage locking mechanism 60b has a detent bias hook 102b bearing against the lever pin 84 to hold the lever pin 84 received in the catch recess 90. The detent bias hook 102b is not integral, but is attached to and separable from the housing 16. In this embodiment the detent bias 102a is a spring hook on a swivel pin. In another example illustrated in Fig. 5C, the linkage locking mechanism 60c has a detent bias 102c bearing against the lever pin 84 to hold it received in the catch recess 90a. The detent bias 102c had a flexible finger 103 extending from (and integral to) the housing 16c and crossing over the lever pin 84. A blocking head portion 105 of the detent bias finger 102c provided the force to hold the lever pin 84 against the catch recess 90a. Again, the detent bias 102c bearing against the lever pin 84 was integral to and inseparable from the housing 16c. However, other detent bias means are known to and selectable by one of ordinary skill in the art for practice in the present invention.

Referring to Figures 1A to 4 to illustrate use of this embodiment of the holder 10, a tool head 40, such as a surgical rasp, is introduced into the tool port 38. Pushing down on the handle 88 of the engagement lever 80 causes the torsion connecting rod 70 to move as indicated. This movement is communicated to the locking pawl 65 causing its locking face 68 (see Fig. 3A) to engage a detent 42 in the tool head 40 to secure the tool 40 to the handle 10. The disposition of components of the linkage 50, upon the action of engaging the lever 80, torques the connecting rod 70 over its length causing a constant force to be exerted at both of its ends 72 & 74 when the linkage 50 is configured in its closed configuration. This force holds the locking pawl 65 engaged with the tool detent notch 42, and acts to keep the engagement lever 80 toggled in the closed position. For cleaning and sterilization, the engagement lever 80 is manually released from the linkage lock mechanism 60 and the tool holder linkage 50 swiveled from the linkage chamber 28 to make it accessible for cleaning.

Optionally, in addition to the linkage lock mechanism 60 in this embodiment, a lever lock 110 can be practiced in the present surgical tool holder 10. A lever lock 110 is a means for further preventing the lever handle 88 of the tool holder linkage 50 from being inadvertently operated from the closed configuration (see Fig. 2B). In the embodiment illustrated in Fig. 6, the lever lock 110 comprises the setting of an obstruction 116 closely adjacent the follower arm 82 of the engagement lever 80 to prevent an inadvertent force on the handle lever 88 from causing the engagement lever 80 to rotate. In the embodiment shown, the rotation of a section 114 of the grip 34 causes extension of the obstruction block 116 from its retracted position (see Figs. 1A and 1B) to its locking position. A change in the texture of the hand grip 34 is useful for indicating the configuration of the lever lock 110 - retracted or extended. Mechanisms for accomplishing the movement of the block 116 by rotation of sections of the hand grip 34 relative to each other are known in the art, and are selectable by the ordinary skill artisan and accomplished in the present invention.

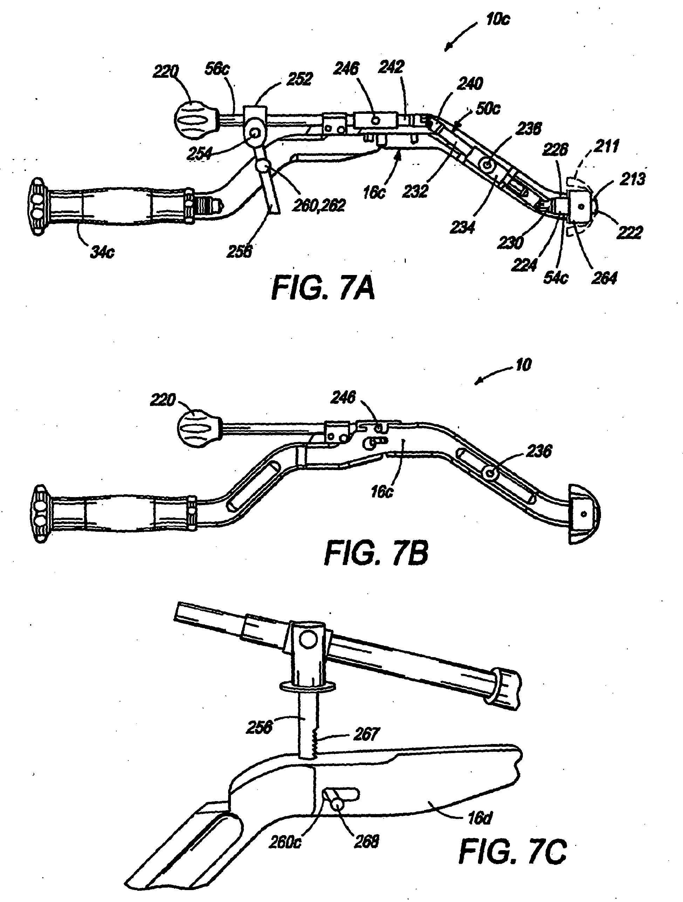

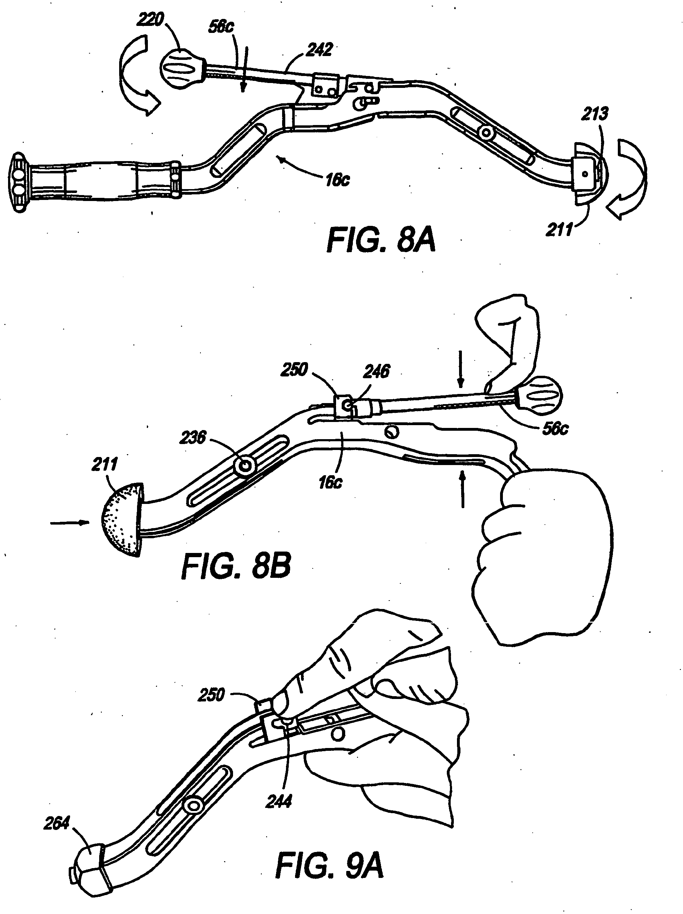

The tool holder linkage assembly 50 of the present invention can structurally and functionally be more than just a means for attaching a tool head to the holder 10. In another preferred embodiment illustrated Fig. 7A and subsequent, the break-away tool holder linkage assembly 50 comprised a tool head drive chain 50c. In use, the tool head drive chain 50c is retained in large part within the housing 16c. An attachment 211, such as a surgical tool, a surgical appliance or a prosthetic element, is installable to the tool end 54c of the drive chain 50c at a tool interface 213. At the retainer end 56c of the drive chain 50c a handle 220 is fixed to the pivoting lever 242 of the drive chain 50c. Rotation of the handle 220 by a user is transmitted via the drive chain 50c and imparted to the attachment 211 (see Fig. 8A). In the embodiment illustrated, the holder 10c is configured to aid a surgeon in controlling the installation of an acetabular cup prosthesis. The housing 16c was C-shaped, as shown, in order to minimize the invasiveness of the surgery by better clearing anatomical structures and tissue.

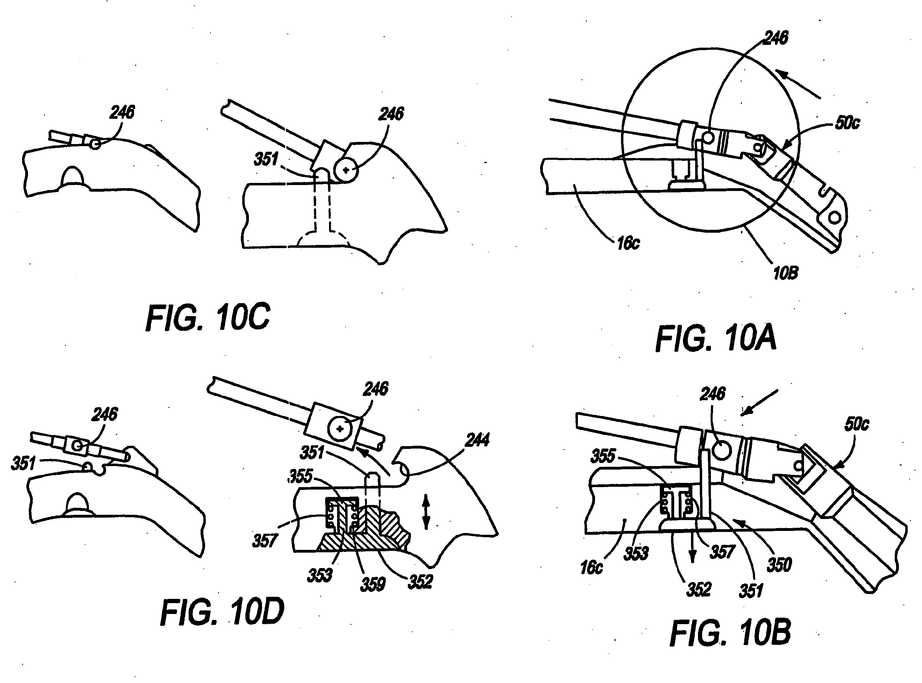

The interface 213 was cut on a boss 222 on a cylindrical piston 224 which slides in an axial hole 226 in the housing head 264 of the housing 16c. The interface 213 is preferably threaded. In the preferred embodiment illustrated, the head 264 was a polymeric impactor head molded over the tool end 24 of the housing 16c. A molded polymeric head is useful to absorb impact stresses incurred during use of the present tool holder 10 as a surgical impactor. The housing head 264 is also selected so as to have good frictional characteristics as well. Nevertheless, a metal, non-molded housing head 264 may also be used with satisfactory results. The piston 224 was connected by way of a first U-joint 230 to a lever 232 which slides in a pivoting sleeve 234 fixed to the housing 16c via a pivot 236. The lever 232 is connected via a second U-joint 240 to the second pivoting lever 242 which was fixed to pivot in a catch 244 (see Fig. 10D) on a pivot pin 246. The catch 244 is essentially a seat or detent cut into the housing 16c, against which the pivot pin 246 of the lever 242 is captured when a slide 250 (see Fig. 8B) is slid over the pivot pin 246 when it is engaged against the catch 244.

The lever 242 has a slideable sleeve 252, through which it passes. The lever 242 has a trunnion 254 to which a rod 256 is pivotally attached. The rod 256 passes through a one-way catch 260 in the housing 16c. The one-way catch 260 prevents the rod 256 from sliding out of the housing 16c, unless a release (not shown) is operated. The one-way catch 260 can be a captured split wedge sleeve 262 having an inner diameter that just matches the outer diameter of the rod 256, and which is captured in a recess having a matching conical surface that surrounds the sleeve so as to allow the rod 256 to slide into the housing 16c, but to prevent the rod from sliding out, the release is operated. The release could be a lever for merely lifting the sleeve 262 out of engagement with the conical surface so as to unlock and to permit the rod 256 to back out of the housing 16c. However, this is just an example, and any number of alternative one-way lock devices may be used, the selection of which being within the skill of a person of ordinary skill in this field. For example, Fig. 7C illustrates an alternative embodiment of a one-way catch mechanism 260. In this embodiment, the rod 256c passes through a one-way catch 260c in the housing 16d. The one-way catch 260c has an inner recess that matches the quter diameter of the rod 256c. The inner recess has a ratchet pawl (not shown) that locks against one-way ratchet teeth 267 so as to allow the rod 256c to slide into the housing 16d, but to prevent the rod 256c from sliding out, unless a release lever 268 is operated. In the example illustrated, release is accomplished by operating the lever 268 to merely pull the pawl away from the teeth 267 to permit the rod 256c to back out of the housing 16d.

Referring now to Figs. 8A and 8B, in operation the interface (preferably threaded) of the piston 224 is engaged with the prosthesis 211. When the operator rotates the handle 220 about the axis of the pivoting lever 242, this rotates the drive train 50c which in turn rotates the piston 224. Rotation of the piston 224 can be used to attach the interface 213 to an attachment 211, to impart rotational force to an attachment 211 already engaged in the interface 213, or to orient the attachment 211 in desired position. This last feature is particularly useful when the attachment is a prosthesis being installed in a bone site.

As illustrated in Fig. 8B, when the retainer end 56c of the drive lever 242 is urged downwardly toward the housing 16c, movement is transmitted through the drive train 50c to draw the piston 24 into the housing 12, and thus to cause the attachment 211 to be drawn against the housing head 264 of the housing 16c. This contact serves to create a normal force between the attachment 211 and the head 264 so as to prevent rotation of the attachment 211 relative to the housing 16c. The operator may use the one way locking mechanism 262 to lock the lever 242 in a position so as to lock the attachment 211 against the housing head 264. One of the benefits of this feature is that the operator can pre-set and lock the position of the attachment 211 prior to engaging it with the bone site. Note that this feature is particularly beneficial when the attachment 211 is a prosthetic device. This is because a prosthesis often has pre-drilled holes, which must be properly positioned prior to fastening through these at the installation site.

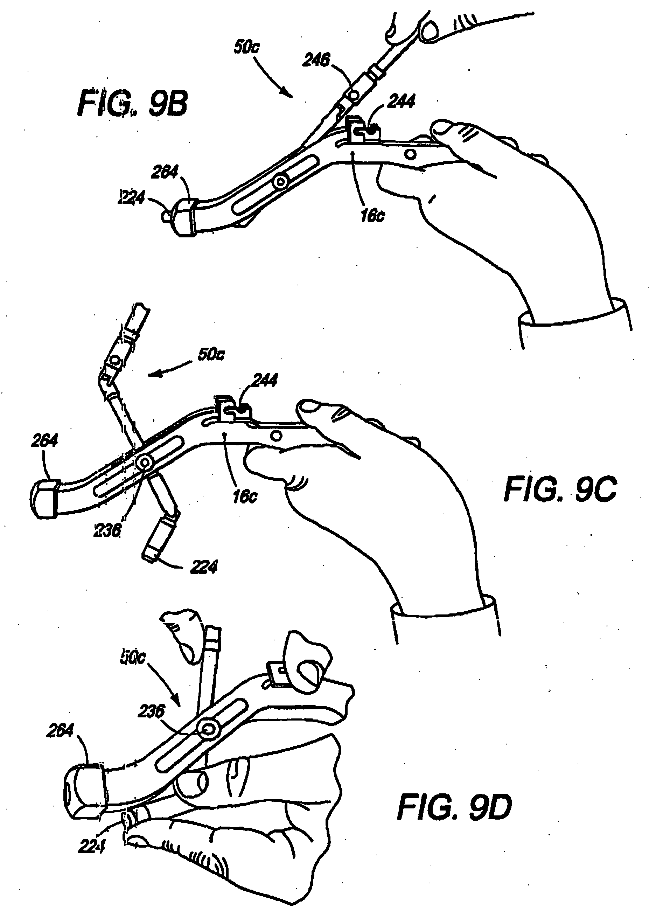

The "easily cleaned" feature of the present invention enables access to all surfaces, so that they can be cleaned, i.e., parts covering other parts can be moved to expose all surfaces for cleaning and sterilization. Also, the present invention is practiced with a reduction in number of small radius internal corners, crevices and small gaps, and the absence of blind holes. Referring now to Figs. 9A to 9D, in the embodiment shown, the device 10 is disassembled for cleaning by simply sliding the slide 250 back to release the pivot 246 and then folding the drive train 50c out of the housing 16c. However, the drive chain 50c remains tethered to the housing 16c by the pivot pin 236. As the drive train 50c is swung out of the housing 16c, the piston 224 is drawn out of the hole 226 in the housing 16c. To reassemble holder after cleaning, the piston 224 is reinserted into the hole 226 and the drive train 50c is swung back into housing 16c, with the one-way locking mechanism entering its receiver and the pivot 246 again entering into the catch 244. The slide 250 is then slid over the pivot2 46 and the present surgical tool holder 10 is again ready for use.

Referring now to Figs. 10A and 10B, an alternate embodiment of the present surgical tool holder 10 is shown. In this embodiment, the tool holder 10 has a safety release 350 having a safety release pin 351 connected to a base plate 352. Also connected to the base plate is a second spring pin 253 which is spaced apart and parallel with the release pin 351. The second spring pin 353 has a boss 355 which retains a safety release spring 357. The release spring 357 acts between the boss 355 and a release spring seat surface 359 of the housing 16d to hold the safety release in a normally closed condition by biasing the release pin 351 upward in a position which holds the cross pin 246 of the drive train 50c against the catch 244. Preferably, the release pin 351 has a rounded head 361 to facilitate removal of a pivot pin 246 from its respective catch 244 and allowing disassembly of the tool holder 10 for cleaning/sterilization. Further, the overall normal bias force of the safety release 350 is selected so as to release the pivot pins 246 from their respective catches 244 when the torsion or the stress on the drive train 50c (for example, from use of the holder 10 as an impactor) reaches a certain amount that, if exceeded, might damage the drive train 50c. Typically, such stresses occur when there is no tension on the drive train 50c and the pivot pins 246 collide with the safety release pin 351. Using this embodiment, it is also possible to release the pivot pin 246 from the 244 by the operator pulling down on a portion of the plate 252 that extends beyond the housing 16d. Release of the pivot pins 246 from the catches 244 is further shown in Figs. 10C and 10D.

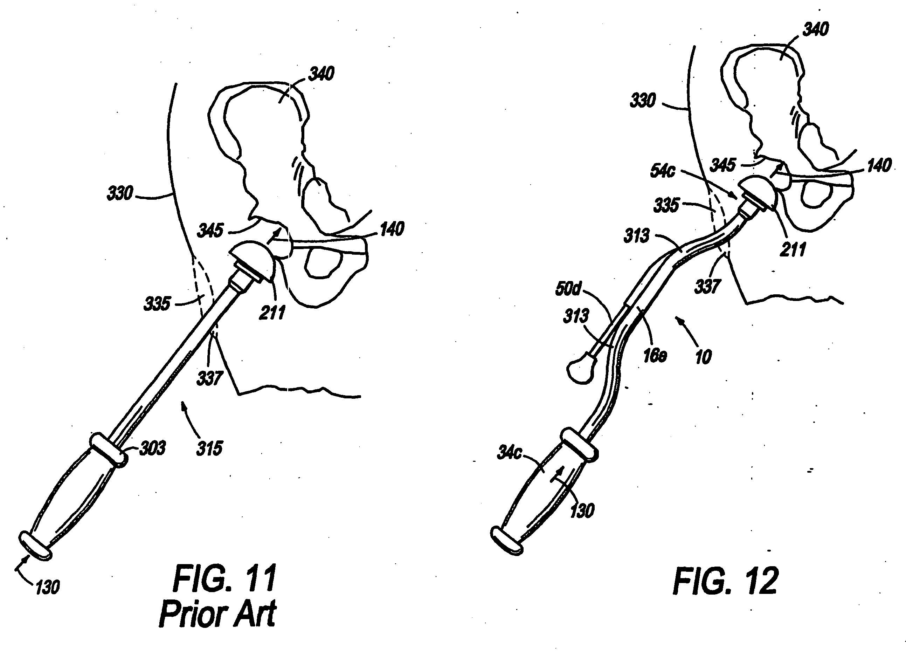

Figs. 11 and 12 represent a prior art holder 315 and the present surgical tool holder 10 respectively, passing through an incision 335 in a patient's skin 330. Fig. 12 shows the preferred embodiment of the present tool holder 10 which has an appropriately curved housing 16e containing the drive chain 50d. The figures illustrate the benefit of a "C"-shaped housing 16e, in that in Fig. 12 the tool holder 10 is shown approaching the acetabulum 340 in an orientation desirable to ream the socket 345. A difficulty with the prior art "spindle"-type tool holder 315 in Fig. 11 is shown as the shaft 303 impinging on the miniature incision 335 at edge of the incision 337. Current surgical protocols are being pushed to the limits, with incision sizes being reduced to increase the patient's recovery speed. In some cases, surgeons are using a two-incision approach, one to reach the acetabulum and the other to reach the femur. Both the one incision and the two incision technique demand less trauma to the patient, requiring the instruments to be more optimally designed to make up for the lack of operating space.

It is important to place the bends or off-sets 313 in the housing 16e at critical locations to pass through a miniature incision 335 without impinging on the skin at the edge 337 of the incision, while still maintaining the miniature incision surgical protocol. The bends/off-sets 313 are further disposed so that the grip end 34c of the holder 10 and the tool holder end 54c of the drive chain 50c have parallel axes, so that an applied force 130 on the grip end 34c results in an axial motion 140 at the tool holder end 54c. This configuration allows the operator to maintain the desired miniature incision technique using a curved housing embodiment of the present tool holder 10, without the above noted difficulty inherent in the use of the prior inserter 315 of Fig. 11. Additionally, in use, a curved housing embodiment of the present tool holder 10 provides the same benefit as a prior straight "spindle"-type tool holder of allowing the surgeon to apply a load directly along the path of reaming.

It is inherent in the present surgical tool holder 10 that alternative housing heads 264 can be mounted onto the front of the tool holder 10, which alternative heads 264 conform with a surface of specific alternative attachment 211, such as, an acetabular cup liner, in order to enable the device to seat a liner as well as the acetabular cup illustrated. In an advantage, the present tool handle 10 is simple and easy to use, without complex and possibly confusing locks. It is an object of the present invention is to minimize the risk that parts could be lost by having the holder 10 be partially disassemblable for cleaning and sterilization, but having all component parts remaining linked together.

Multiple variations and modifications are possible in the embodiments of the invention described here. Although certain illustrative embodiments of the invention have been shown and described here, a wide range of modifications, changes, and substitutions is contemplated in the foregoing disclosure. In some instances, some features of the present invention may be employed without a corresponding use of the other features. Accordingly, it is appropriate that the foregoing description be construed broadly and understood as being given by way of illustration and example only, the scope of the invention being limited only by the appended claims.

## Claims

1. A surgical orthopedic tool holder (10) comprising a housing (16) with a proximal grip (34) and a linkage chamber (28) for receiving a tool holder linkage (50), the tool holder linkage being fixed to the housing by a locking means (66, 84, 90, 60), **characterized in that** the linkage is pinned at a point in the chamber such that when the locking means is released, the tool holder linkage is pivotable out of the chamber for cleaning.

2. The surgical tool holder (10) of claim 1, the holder having a lockable, tethered tool holder linkage (50) providing for facilitated cleaning and sterilization, the tool holder (10) comprising:
the housing (16) being elongated and having a grip end (20), a tool end (24), and a linkage chamber (28), with a hand grip (34) attached to the housing (16) at the grip end (20) and a surgical tool interface (64) disposed at the tool end (24);
the tool holder linkage (50) having the tool holder end (54) and a linkage retainer end (56), with the tool holder linkage (50) being receivable into the linkage chamber (28) with the tool holder linkage (50) tethered at the tool holder end (54) to the linkage chamber (28) by a pivot pin (66), and the linkage retainer end (56) having a pair of opposed lever pins first mating means (84) releaseably engageable with a pair of catch recesses (90) of a linkage lock mechanism (60); and
the linkage lock mechanism (60) proximate the grip end (20) of the housing (16), the linkage lock mechanism (60) engageable to lock the tool holder linkage (50) in the linkage chamber (28) during use, and reieaseable to allow the tool holder linkage (50) to pivot out facilitate cleaning and sterilization to the tool holder (10).

3. The surgical tool holder (10) of claim 1, wherein, the tool holder linkage (50) at the tool holder end (54) has a locking pawl (65) cooperating with the pivot pin (66), the locking pawl (65) in driven communication with a driven end (72) of a torsion connecting rod (70), and the torsion connecting rod (70) having a drive end (74) in driven communication with a lever follower arm (82) of an engagement lever (80) first mating means at the linkage retainer end (56) of the tool holder linkage (50).

4. The surgical tool holder (10) of claim 3, wherein:
the engagement lever (80) has a pair of opposed lever pins (84) centered on an engagement lever axis (86);
the elongated housing (16) including a pair of catch recesses (90) proximate the grip end (20), each catch recess (90) disposed to pivotably receive a lever pin (84) of the pair of lever pins to mount the engagement lever (80), and the engagement lever (80) being pivotable about the lever axis (86) when mounted in the catch recess (90) so that moving a handle end (58) of the engagement lever (80) brings the follower arm (82) to bear against the drive end (74) of the torsion connecting rod (70); and
the second mating means (90) of the linkage lock mechanism (60) engages at least one of the lever pins (84) of the engagement lever (80) to retain the tool holder linkage (50) in the linkage chamber (28).

5. The surgical tool holder (10) of claim 4, wherein the second mating means (90) of the linkage lock mechanism (60) comprises a detent bias means (102) bearing against at least one of the lever pins to hold the lever pin received in the catch recess.

6. The linkage lock mechanism of claim 5, wherein the detent bias means (102) is integral to and inseparable from the housing (16).

7. The surgical tool holder (10) of claim 4, wherein the linkage lock mechanism (60) comprises a retractable obstruction (116) normally blocking at least one of the catch recesses (90) to prevent removal of the lever pin (84) received therein and acting to retain the lever pin (84) received in the catch recess (90).

8. The surgical tool holder (10) of claim 3, further comprising a lever lock (110) for selectively preventing the engagement lever (80) of the tool holder linkage (50) from being inadvertently operated from a closed configuration.

## Patentansprüche

1. Halter (10) für ein chirurgisches Orthopädieinstrument, das ein Gehäuse (16) mit einem proximalen Griff (34) und einer Gestängekammer (28) zur Aufnahme eines Instrumentenhaltergestänges (50) aufweist, wobei das Instrumentenhaltergestänge durch ein Verriegelungsmittel (66, 84, 90, 60) am Gehäuse befestigt ist, **dadurch gekennzeichnet, dass** das Gestänge so an einer Stelle in der Kammer gelenkig verbunden ist, dass das Instrumentenhaltergestänge zum Reinigen aus der Kammer geschwenkt werden kann, wenn das Verriegelungsmittel gelöst wird.

2. Halter (10) für ein chirurgisches Instrument nach Anspruch 1, wobei der Halter ein verriegelbares, angebundenes Instrumentenhaltergestänge (50) hat, das für leichteres Reinigen und Sterilisieren sorgt, wobei der Instrumentenhalter (10) Folgendes aufweist:
das Gehäuse (16), das länglich ist und ein Griffende (20), ein Instrumentenende (24) und eine Gestängekammer (28) hat, wobei ein Handgriff (34) am Griffende (20) am Gehäuse (16) angebracht ist und
eine Schnittstelle (64) für das chirurgische Instrument am Instrumentenende (24) angeordnet ist,
das Instrumentenhaltergestänge (50), das das Instrumentenhalterende (54) und ein Gestängehalterende (56) aufweist, wobei das Instrumentenhaltergestänge (50) in der Gestängekammer (28) aufgenommen werden kann, wobei das Instrumentenhaltergestänge (50) über einen Schwenkstift (66) am Instrumentenhalterende (54) an die Gestängekammer (28) angebunden ist und das Gestängehalterende (56) ein erstes Passmittel eines Paars gegenüberliegender Hebelstifte (84) hat, das mit einem Paar Rastausnehmungen (90) eines Gestängeverriegelungsmechanismus (60) lösbar in Eingriff gebracht werden kann, und
den Gestängeverriegelungsmechanismus (60) in der Nähe des Griffendes (20) des Gehäuses (16), der in Eingriff gebracht werden kann, um das Instrumentenhaltergestänge (50) während des Gebrauchs in der Gestängekammer (28) zu verriegeln, und ausgerückt werden kann, damit das Instrumentenhaltergestänge (50) herausschwenken kann, um die Reinigung und Sterilisierung des Instrumentenhalters (10) zu erleichtern.

3. Halter (10) für ein chirurgisches Instrument nach Anspruch 1, wobei das Instrumentenhaltergestänge (50) am Instrumentenhalterende (54) eine mit dem Schwenkstift (66) zusammenwirkende Verriegelungsklinke (65) hat, die in angetriebener Verbindung mit einem angetriebenen Ende (72) einer Torsionsverbindungsstange (70) steht, die ein Antriebsende (74) hat, das am Gestängehalteende (56) des Instrumentenhaltergestänges (50) in angetriebener Verbindung mit einem Hebelfolgerarm (82) eines ersten Passmittels des Eingriffshebels (80) steht.

4. Halter (10) für ein chirurgisches Instrument nach Anspruch 3, wobei
der Eingriffshebel (80) ein Paar gegenüberliegende, auf eine Eingriffshebelachse (86) zentrierte Hebelstifte (84) hat,
das längliche Gehäuse (16) ein Paar Rastausnehmungen (90) in der Nähe des Griffendes (20) aufweist, wobei jede Rastausnehmung (90) so angeordnet ist, dass sie einen Hebelstift (84) des Paars Hebelstifte schwenkbar aufnimmt, um den Eingriffshebel (80) zu montieren, und wobei der Eingriffshebel (80) um die Hebelachse (86) schwenkbar ist, wenn er in der Rastausnehmung (90) montiert ist, so dass der Folgerarm (82) durch Bewegen eines Handgriffendes (58) des Eingriffshebels (80) am Antriebsende (74) der Torsionsverbindungsstange (70) zur Anlage kommt, und
das zweite Passmittel (90) des Gestängeverriegelungsmechanismus (60) mindestens einen der Hebelstifte (84) des Eingriffshebels (80) in Eingriff nimmt, um das Instrumentenhaltergestänge (50) in der Gestängekammer (28) zu halten.

5. Halter (10) für ein chirurgisches Instrument nach Anspruch 4, wobei das zweite Passmittel (90) des Gestängeverriegelungsmechanismus (60) ein Arretierungsvorspannungsmittel (102) aufweist, das an mindestens einem der Hebelstifte anliegt, um den in der Rastausnehmung aufgenommenen Hebelstift zu halten.

6. Gestängeverriegelungsmechanismus nach Anspruch 5, wobei das Arretierungsvorspannungsmittel (102) einstückig und untrennbar vom Gehäuse (16) ist.

7. Halter (10) für ein chirurgisches Instrument nach Anspruch 4, wobei der Gestängeverriegelungsmechanismus (60) ein zurückziehbares Hindernis (116) aufweist, das normalerweise mindestens eine der Rastausnehmungen (90) blockiert, um die Entfernung des darin aufgenommenen Hebelstifts (84) zu verhindern, und so wirkt, dass der Hebelstift (84) in der Rastausnehmung (90) aufgenommen wird.

8. Halter (10) für ein chirurgisches Instrument nach Anspruch 3, ferner mit einer Hebelverriegelung (110), um gezielt zu verhindern, dass der Eingriffshebel (80) des Instrumentenhaltergestänges (50) versehentlich aus einer geschlossenen Konfiguration betätigt wird.

## Revendications

1. Porte-instrument chirurgical orthopédique (10) comportant un boîtier (16) muni d'un manche proximal (34) et d'une chambre (28) de tringlerie destinée à recevoir une tringlerie (50) de porte-instrument, la tringlerie de porte-instrument étant fixée au boîtier par un moyen (66, 84, 90, 60) de blocage **caractérisé en ce que** la tringlerie est goupillée au niveau d'un point de la chambre de telle sorte que, lorsque le moyen de blocage est relâché, la tringlerie de porte-instrument puisse pivoter hors de la chambre en vue d'un nettoyage.

2. Porte-instrument chirurgical (10) selon la revendication 1, le porte-instrument comprenant une tringlerie (50) de porte-instrument verrouillable et ancrée installée pour un nettoyage et une stérilisation facilités, le porte-instrument (10) comportant :
le boîtier (16) qui est allongé et présente une extrémité (20) côté manche, une extrémité (24) côté instrument et une chambre (28) de tringlerie, un manche (34) de préhension manuelle étant fixé au boîtier (16) au niveau de l'extrémité (20) côté manche et une interface (64) d'instrument chirurgical étant disposée à l'extrémité (24) côté instrument ;
la tringlerie (50) de porte-instrument présentant l'extrémité (54) côté porte-instrument et une extrémité (56) de retenue de tringlerie, la tringlerie (50) de porte-instrument pouvant être logée dans la chambre (28) de tringlerie avec la tringlerie (50) de porte-instrument ancrée au niveau de l'extrémité (54) côté porte-instrument à la chambre (28) de tringlerie par une goupille (66) formant pivot, et l'extrémité (56) de retenue de tringlerie étant munie d'une paire de premiers moyens (84) d'accouplement à goupilles de levier opposées, susceptibles de s'enclencher de façon détachable avec une paire d'évidements (90) de coincement d'un mécanisme (60) de blocage de tringlerie ; et
le mécanisme (60) de blocage de tringlerie voisin de l'extrémité (20) côté manche du boîtier (16), le mécanisme (60) de blocage de tringlerie pouvant être enclenché pour bloquer la tringlerie (50) de porte-instrument dans la chambre (28) de tringlerie en cours d'utilisation, et pouvant être relâché pour permettre à la tringlerie (50) de porte-instrument de pivoter vers l'extérieur pour faciliter le nettoyage et la stérilisation du porte-instrument (10).

3. Porte-instrument chirurgical (10) selon la revendication 1, la tringlerie (50) de porte-instrument comportant, à l'extrémité (54) côté porte-instrument, un linguet (65) de blocage coopérant avec la goupille (66) formant pivot, le linguet (65) de blocage étant en communication de transmission avec une extrémité (72) menée d'une tige (70) de liaison en torsion et la tige (70) de liaison en torsion présentant une extrémité menante (74) en communication de transmission avec un bras suiveur (82) de levier d'un premier moyen d'accouplement de levier (80) d'enclenchement à l'extrémité (56) de retenue de tringlerie de la tringlerie (50) de porte-instrument.

4. Porte-instrument chirurgical (10) selon la revendication 3,
le levier (80) d'enclenchement comportant une paire de goupilles (84) de levier opposées centrées sur un axe (86) de levier d'enclenchement ;
le boîtier allongé (16) comprenant une paire d'évidements (90) de coincement à proximité de l'extrémité (20) côté manche, chaque évidement (90) de coincement étant disposé de façon à recevoir une goupille (84) de levier de la paire de goupilles de levier pour monter le levier (80) d'enclenchement, et le levier (80) d'enclenchement pouvant pivoter autour de l'axe (86) de levier lorsqu'il est monté dans l'évidement (90) de coincement, de sorte que le fait de déplacer une extrémité (58) côté manche du levier (80) d'enclenchement amène le bras suiveur (82) à s'appuyer contre l'extrémité menante (74) de la tige (70) de liaison en torsion ; et
le deuxième moyen (90) d'accouplement du mécanisme (60) de blocage de tringlerie coopérant avec au moins une des goupilles (84) de levier du levier (80) d'enclenchement pour retenir la tringlerie (50) de porte-instrument dans la chambre (28) de tringlerie.

5. Porte-instrument chirurgical (10) selon la revendication 4, le deuxième moyen (90) d'accouplement du mécanisme (60) de blocage de tringlerie comportant un moyen de cran (102) de sollicitation appuyant sur au moins une des goupilles de levier pour maintenir la goupille de levier logée dans l'évidement de coincement.

6. Mécanisme de blocage de tringlerie selon la revendication 5, le moyen de cran (102) de sollicitation étant intégré au boîtier (16) et indissociable de celui-ci.

7. Porte-instrument chirurgical (10) selon la revendication 4, le mécanisme (60) de blocage de tringlerie comportant un obstacle rétractable (116) bloquant normalement au moins un des évidements (90) de coincement pour empêcher le retrait de la goupille (84) de levier qui y est logée et agissant pour retenir la goupille (84) de levier logée dans l'évidement (90) de coincement.

8. Porte-instrument chirurgical (10) selon la revendication 3, comportant en outre un verrou (110) de levier destiné à empêcher sélectivement le levier (80) d'enclenchement de la tringlerie (50) de porte-instrument d'être actionné involontairement à partir d'une configuration fermée.
